# EUROPÄISCHE PATENTSCHRIFT

(11) **EP 3 067 079 B1**
(45) Veröffentlichungstag und Bekanntmachung des Hinweises auf die Patenterteilung: **18.08.2021**
(21) Anmeldenummer: 16157113.8
(22) Anmeldetag: 24.02.2016
(51) Int. Cl.: A61M 5/30, A61M 5/19, A61M 5/32, A61M 5/34, A61M 5/315, A61M 5/20

(54) **SPRITZE MIT EINEM ERSTEN UND ZWEITEN SPRITZENZYLINDER**
SYRINGE WITH A FIRST AND SECOND SYRINGE BARREL
SERINGUE AVEC UN PREMIER ET UN DEUXIÈME CORPS DE SERINGUE

(30) Priorität: 13.03.2015 DE 102015103750
(43) Veröffentlichungstag der Anmeldung: 14.09.2016
(73) Patentinhaber: Henke-Sass, Wolf GmbH, 78532 Tuttlingen (DE)
(72) Erfinder: Eisele, Melanie, 78573 Wurmlingen (DE); Altermann, Frank, 78532 Tuttlingen (DE)
(74) Vertreter: Patentanwälte Geyer, Fehners & Partner mbB

(56) Entgegenhaltungen:
- WO-A1-2012/013587
- WO-A2-03/075978
- None

## Beschreibung

Die vorliegende Erfindung betrifft eine Spritze mit einem ersten und zweiten Spritzenzylinder.

Solche Spritzen werden z.B. im Veterinärbereich eingesetzt, um den Tieren zwei unterschiedliche Medikamente gleichzeitig zu verabreichen. Damit die Spritze einen möglichst großen Anwendungsbereich hat und somit bei unterschiedlichsten Tieren genutzt werden kann, ist es hilfreich, wenn der Abstand der Hohlkanülen, die mit den Spritzenzylindern verbindbar sind, einstellbar ist. Dazu kann im einfachsten Fall der Abstand der Spritzenzylinder und damit auch der Abstand der Kanülen veränderbar vorgesehen sein. Dies führt in der Regel jedoch zu einer aufwendigen Mechanik und zu Schwierigkeiten bei der Stabilität der Spritze.

Die WO 2012/013587 A1 beschreibt eine Nadelanordnung für eine Injektionsvorrichtung, wobei die Nadelanordnung einen Adapter aufweist, in dem zwei Hohlkanülen mit nicht änderbarem Abstand voneinander befestigt sind.

Ausgehend hiervon ist es Aufgabe der Erfindung, eine Spritze mit zwei Spritzenzylindern vorzusehen, bei der die Einstellung des Abstands der Abgabeenden leicht möglich ist.

Die Erfindung ist im Anspruch 1 definiert. Vorteilhafte Weiterbildungen sind in den abhängigen Ansprüchen angegeben.

Da erfindungsgemäß zur Verstellung des Abstandes der Kanäle nur das zumindest eine der beiden Endstücke in unterschiedlichen Stellungen am zugehörigen Abgabeende befestigt werden muss, kann in einfacher Art der gewünschte Abstand eingestellt werden. Gleichzeitig ist die Stabilität der Spritze nicht beeinträchtigt, da der Abstand der Spritzenzylinder selbst nicht änderbar ist und somit die mechanische Verbindung der Spritzenzylinder so dimensioniert werden kann, dass die erforderliche Stabilität sicher bereitgestellt ist.

Die erfindungsgemäße Spritze kann als Veterinärspritze ausgebildet sein. Nachdem die Drehung des Endstücks um die Mittelachse erfolgt, führt die exzentrische Anordnung des Kanals dazu, dass in Abhängigkeit der Drehstellung des Endstücks der Abstand der Kanäle geändert wird.

Bei der erfindungsgemäßen Spritze kann das zumindest eine der beiden Endstücke ein zum zugeordneten Spritzenzylinder zugewandtes Ende aufweisen, das in eine am Abgabeende des zugeordneten Spritzenzylinders vorgesehene Aufnahme in zumindest zwei unterschiedlichen Drehstellungen einsetzbar ist. Insbesondere kann es in den unterschiedlichen Drehstellungen fixiert oder arretiert werden, so dass ein unerwünschtes Ändern der Drehstellung sicher verhindert wird. Zur Fixierung oder Arretierung kann eine Fixiereinrichtung vorgesehen sein.

Die Aufnahme kann insbesondere hohlzylinderförmig und das entsprechende Ende kann zylinderförmig ausgebildet sein. Insbesondere kann die hohlzylinderförmige Ausnehmung eine n-kant Kontur (bzw. eine Kontur mit n Kanten) und das entsprechende Ende eine m-kant Kontur (bzw. eine Kontur mit m Kanten) aufweisen, wobei n und m ganze Zahlen sind und n größer oder gleich m ist. Somit ist es möglich, n verschiedene Drehstellungen vorzugeben, in denen das Endstück befestigt werden kann. Die n- und m-kant Kontur weisen im Querschnitt bevorzugt die Form eines (insbesondere regelmäßigen) Polygons mit n- bzw. m-Ecken auf.

Bei der erfindungsgemäßen Spritze kann das zumindest eine der beiden Endstücke mittels einer Überwurfmutter am zugeordneten Abgabeende befestigt sein. Damit wird in einfacher Art und Weise die gewünschte Fixierung bereitgestellt.

Insbesondere können die beiden Endstücke so ausgebildet sein, dass an ihnen lösbar jeweils eine Hohlkanüle befestigbar ist.

Bei der Spritze können die beiden Spritzenzylinder zueinander parallel ausgerichtet sein. Des Weiteren können die beiden Kanäle zueinander parallel ausgerichtet sein.

Die Spritze ist insbesondere als Selbstfüllerspritze ausgebildet.

Die Spritze kann ein Hauptteil aufweisen, an dem die beiden Spritzenzylinder mechanisch befestigt sind. An dem Hauptteil kann ein Hebel angelenkt sein, mit dem die Kolben in die beiden Spritzenzylinder (beispielsweise über Kolbenstangen) hin und her bewegt werden können. Insbesondere kann der Hebel an einem von den Spritzenzylindern abgewandten Ende des Hauptteils angelenkt sein. Der Hebel und das Hauptteil können durch eine Feder vorgespannt sein. Die Spritze kann insbesondere pistolenartig ausgebildet sein, so dass ein Benutzer das Hauptteil mit seinen vier Fingern und den Hebel mit seinem Daumen umgreifen kann und durch Schließen der Hand dann den Hebel in Richtung zu den Abgabeenden hin bewegen kann, um das gewünschte Ausspritzen durchzuführen.

Bei der erfindungsgemäßen Spritze kann in zumindest einem der beiden Kolben ein Zuführkanal ausgebildet sein, der in den ersten bzw. zweiten Spritzenzylinder mündet. Über den Zuführkanal kann der entsprechende Spritzenzylinder mit dem abzugebenden Fluid gefüllt werden.

Bei der erfindungsgemäßen Spritze kann der erste Kolben mit einer ersten Kolbenstange verbunden sein, die über das dem ersten Abgabeende abgewandte hintere Ende des ersten Spritzenzylinders heraussteht, der zweite Kolben mit einer zweiten Kolbenstange verbunden sein, die über das dem zweiten Abgabeende abgewandte hintere Ende des zweiten Spritzenzylinders heraussteht, wobei sich der Zuführkanal des zumindest einen der beiden Kolben (oder auch beider Kolben) durch die damit verbundene Kolbenstange erstreckt. Über den Zuführkanal kann das abzugebende Fluid in den Spritzenzylinder gefüllt werden.

Ferner kann der in der Kolbenstange verlaufende Zuführkanal am von dem verbundenen Kolben abgewandten Ende in einen Anschluss münden, in dem ein erstes Rückschlagventil angeordnet ist, das bei einer Bewegung des Kolbens weg vom Abgabenende öffnet. Der Anschluss ist bevorzugt mit einem Reservoir des abzugebenden Fluides verbunden.

Ferner kann die erfindungsgemäße Spritze in zumindest einem der beiden Abgabeenden ein zweites Rückschlagventil aufweisen, das bei einer Bewegung des entsprechenden Kolbens zum Abgabeende hin öffnet.

Die Spritze kann teilweise oder vollständig aus Kunststoff hergestellt sein.

Die Spritzenzylinder und/oder die Endstücke können gleich ausgebildet sein. Es ist jedoch auch möglich, dass sie sich unterscheiden. Beispielsweise kann der Durchmesser der Spritzenzylinder unterschiedlich sein.

Des Weiteren kann die erfindungsgemäße Spritze an jedem Abgabeende eine Injektionskanüle oder Hohlkanüle aufweisen.

Bei der erfindungsgemäßen Spritze kann der erste Kanal in Fluidverbindung mit dem ersten Spritzenzylinder und der zweite Kanal in Fluidverbindung mit dem zweiten Spritzenzylinder stehen. Insbesondere ist jeweils ein Adapter zwischen dem ersten/zweiten Kanal und dem ersten/zweiten Spritzenzylinder vorgesehen.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine perspektivische Ansicht einer Ausführungsform der erfindungsgemäßen Spritze;
- Fig. 2: eine Seitenansicht der Spritze von Fig. 1;
- Fig. 3: eine vergrößerte Schnittansicht entlang der Schnittlinie A-A von Fig. 2;
- Fig. 4: eine Schnittansicht entlang der Schnittlinie B-B von Fig. 3, und
- Fig. 5 bis 8: schematische Darstellungen zur Erläuterung von unterschiedlichen Drehstellungen der Endstücke bezogen auf den Spritzzylinder.

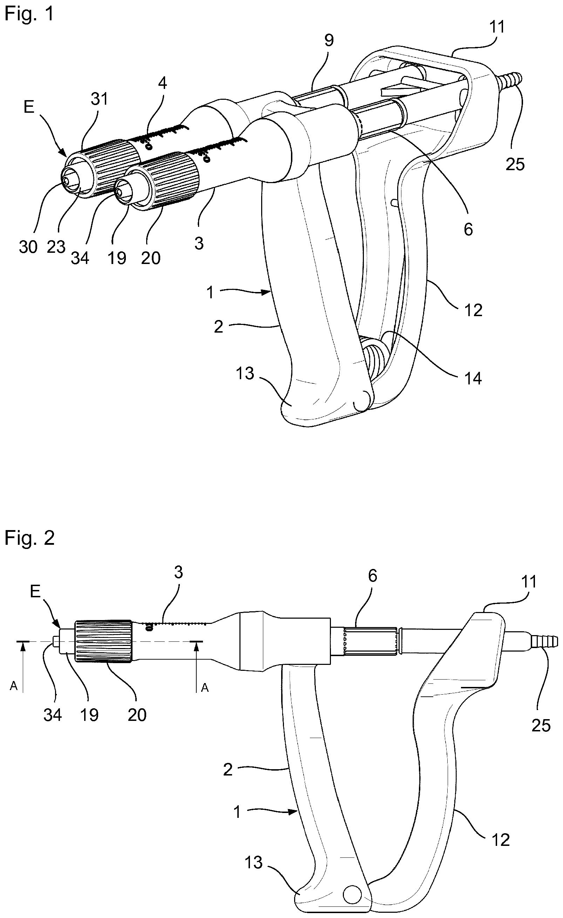

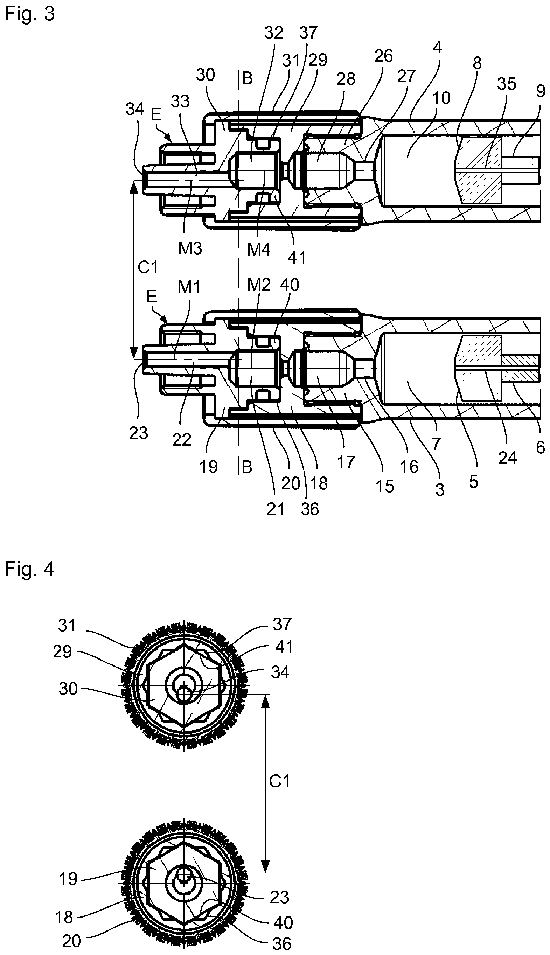

Bei der in Fig. 1 und 2 gezeigten Ausführungsform umfasst die erfindungsgemäße Spritze 1 ein Hauptteil 2, an dem ein erster und ein zweiter Spritzenzylinder 3, 4 ausgebildet sind. Wie insbesondere der Schnittansicht in Fig. 3 zu entnehmen ist, ist im ersten Spritzenzylinder 3 ein erster Kolben 5 angeordnet, der über eine mit dem ersten Kolben 5 verbundene erste Kolbenstange 6 im ersten Spritzenzylinder 3 bzw. im durch den ersten Spritzenzylinder 3 bereitgestellten ersten Spritzenhohlraum 7 hin und her bewegt werden kann. Der zweite Spritzenzylinder 4 ist in gleicher Weise wie der erste Spritzenzylinder 3 ausgebildet und weist einen zweiten Kolben 8 auf, der über eine zweite Kolbenstange 9, die mit dem zweiten Kolben 8 verbunden ist, im durch den zweiten Spritzenzylinder 4 bereitgestellten zweiten Spritzenhohlraum 10 hin und her bewegt werden kann.

Die beiden Kolbenstangen 6, 9 sind über die hinteren (nicht gezeigten) Enden der Spritzenhohlräume 7, 10 herausgeführt und mit einem oberen Ende 11 eines schwenkbar mit dem Hauptteil 2 verbundenen Hebels 12 verbunden (Fig. 1, 2). Der Hebel 12 ist am von den Spritzenzylindern 3, 4 wegweisenden unteren Ende 13 schwenkbar mit dem Hauptteil 2 verbunden, wobei eine im Bereich des unteren Endes 13 angeordnete Feder 14 das Hauptteil 2 und den Hebel 12 auseinanderdrückt, so dass die in Fig. 1 und 2 gezeigte Grundstellung vorliegt. Wenn der Hebel 12 gegen die Kraft der Feder 14 zum Hauptteil 2 hin bewegt wird, werden damit die beiden Kolbenstangen 6 und 9 und somit die beiden Kolben 5 und 8 zum ausspritzseitigen Ende E der Spritze 1 bewegt. In der Schnittdarstellung von Fig. 3 führt dies zu einer Bewegung der Kolben 5, 9 nach links.

Wie insbesondere der Schnittdarstellung in Fig. 3 entnommen werden kann, weist der erste Spritzzylinder 3 an seinem Abgabeende 15 einen ersten Verbindungskanal 16 auf, der den ersten Spritzenhohlraum 7 mit einer ersten Ventilkammer 17 verbindet. Am Abgabeende 15 ist ein erster Adapter 18 montiert (beispielsweise aufgeschraubt), in dem ein erstes Endstück 19 sitzt, das seinerseits mittels einer ersten Überwurfmutter 20 am ersten Adapter 18 befestigt ist. Das erste Endstück 19 weist einen ersten Zwischenraum 21 auf, der über den ersten Adapter 18 in Verbindung mit der ersten Ventilkammer 17 steht. Der erste Zwischenraum 21 mündet in einen ersten Kanal 22 im ersten Endstück 19 und der erste Kanal 22 weist eine erste Abgabeöffnung 23 auf. Somit kann ein Fluid, das im ersten Spritzenhohlraum 7 zwischen dem ersten Kolben 5 und dem ersten Abgabeende 15 vorhanden ist, durch Bewegung des ersten Kolbens 5 zum ersten Abgabeende 15 hin über den ersten Verbindungskanal 16, die erste Ventilkammer 17, den ersten Adapter 18, den ersten Zwischenraum 21 und den ersten Kanal 22 nach außen abgegeben werden. Wie der Schnittdarstellung in Fig. 3 zu entnehmen ist, sind der erste Spritzenhohlraum 7, der erste Verbindungskanal 16, die erste Ventilkammer 17 und der erste Zwischenraum 21 koaxial zueinander angeordnet. Der erste Kanal 22 ist hingegen nicht koaxial zum ersten Zwischenraum 21 und zum ersten Spritzenhohlraum 7 angeordnet, sondern zur entsprechenden Mittelachse M1 des ersten Endstücks 19 versetzt. Die Mittelachse M1 des ersten Kanals 22 ist bevorzugt, wie in Fig. 3 gezeigt ist, parallel zur Mittelachse M2 des ersten Endstücks 19. Ferner kann die Mittelachse M1 des ersten Kanals 22 parallel zur Mittelachse des ersten Spritzenhohlraums 7 bzw. des ersten Zwischenraums 21 sein.

Die Spritze 1 ist als Selbstfüllerspritze ausgebildet und weist dazu in der ersten Ventilkammer 17 ein nicht dargestelltes Rückschlagventil auf, das bei einer Bewegung des ersten Kolbens 5 von links nach rechts (also vom ersten Abgabeende 15 weg) den ersten Verbindungskanal 16 verschließt und bei entgegengesetzter Bewegung des ersten Kolbens 5 (also zum ersten Abgabeende 15 hin) öffnet. Im ersten Kolben 5 und der ersten Kolbenstange 6 ist ein erster Zuführkanal 24 ausgebildet, der sich bis zu einem am hinteren Ende der Spritze 1 angeordneten ersten Anschluss 25 erstreckt, wobei im ersten Anschluss 25 wiederum ein nicht gezeigtes Rückschlagventil angeordnet ist, das bei einer Bewegung des ersten Kolbens 5 vom ersten Abgabeende 15 weg und somit hin zum ersten Anschluss 25 öffnet und bei der entgegengesetzten Bewegung schließt. An dem ersten Anschluss 25 wird während der Benutzung der Spritze 1 ein Fluidreservoir (z.B. ein zu injizierendes Medikament) angeschlossen, so dass bei einer Bewegung des ersten Kolbens 5 vom ersten Abgabeende 15 weg der erste Spritzenhohlraum 7 mit dem Fluid gefüllt wird und bei einer entgegengesetzten Bewegung des ersten Kolbens 5 (also zum ersten Abgabeende 15 hin) das Fluid aus der ersten Abgabeöffnung 23 abgegeben wird oder ausgespritzt wird.

Wie in Fig. 3 gezeigt ist, ist der zweite Kolben 8 baugleich zum ersten Kolben ausgebildet und weist ein zweites Abgabeende 26, einen zweiten Verbindungskanal 27, eine zweite Ventilkammer 28, einen zweiten Adapter 29, ein zweites Endstück 30, eine zweite Überwurfmutter 31, einen zweiten Zwischenraum 32, einen zweiten Kanal 33, eine zweite Abgabeöffnung 34 sowie einen zweiten Zuführkanal 35 auf. Auch ist ein zweiter Anschluss vorgesehen, der jedoch in den Figuren nicht ersichtlich ist. Ferner ist die Mittelachse M3 des zweiten Endstücks 30 in gleicher Weise wie beim ersten Endstück 19 exzentrisch zur Mittelachse M4 des zweiten Kanals 33.

Die beiden Spritzzylinder 3, 4 sind mechanisch so mit dem Hauptteil 2 verbunden, dass ihr Abstand nicht veränderbar ist. Jedoch können die beiden Endstücke 19, 30, wie nachfolgend im Detail beschrieben wird, in verschiedenen Drehstellungen in dem jeweiligen Adapter 18, 29 eingesetzt werden, so dass der Abstand der beiden Abgabeöffnungen 23, 34 variierbar ist.

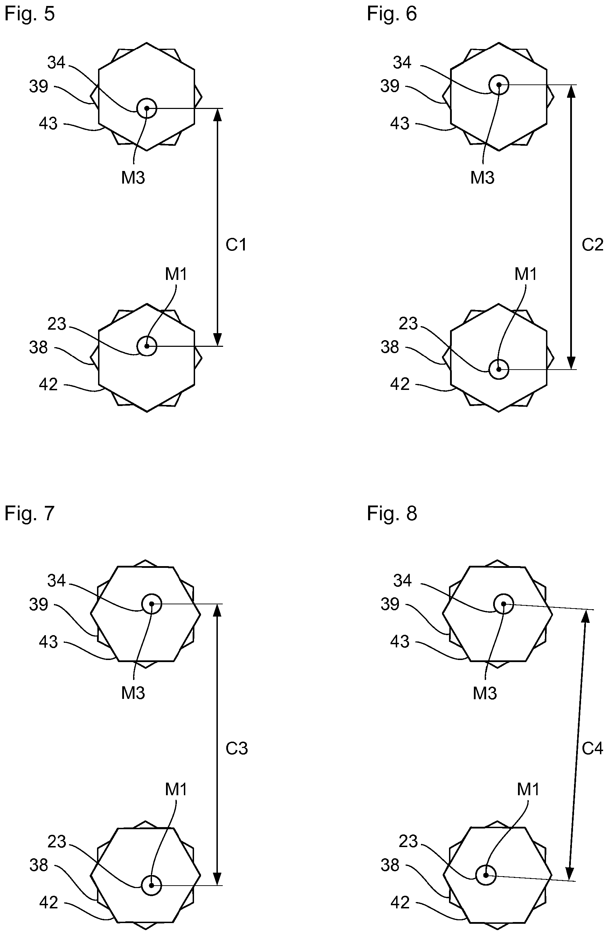

Dazu weist der jeweilige Adapter 18, 29 eine Aufnahme 36, 37 mit einer zwölfkantförmigen Kontur 38, 39 auf. Das in die jeweilige Aufnahme 36, 37 eingesetzte Ende 40, 41 des jeweiligen Endstücks 19, 30 weist jeweils eine sechskantförmige Außenkontur 42, 43 auf, so dass jedes Endstück 19, 30 in zwölf verschiedene Drehstellungen in die zugeordnete Aufnahme 36, 37 eingesetzt und mittels der Überwurfmutter 20, 31 arretiert werden kann. Nachdem die Kanäle 22, 33 nicht koaxial sondern exzentrisch im jeweiligen Endstück 18, 30 angeordnet sind, kann durch Wahl der Drehstellungen der beiden Endstücke 19, 30 der Abstand zwischen den beiden Kanälen 22 und 33 und somit zwischen den beiden Abgabeöffnungen 23 und 34 eingestellt werden. In den Darstellungen von Fig. 3 und 4 ist der minimale Abstand C1 gezeigt, der hier 25 mm beträgt. Zur Verdeutlichung ist in Fig. 5 der gleiche Zustand der beiden Endstücke 19 und 30 wie in Fig. 3 und 4 gezeigt, wobei in der Darstellung von Fig. 5 nur die beiden Konturen 38, 39 sowie 41 und 42, die beiden Abgabeöffnungen 23 und 34 sowie die entsprechende Mittelachse der beiden Kanäle 22 und 33 dargestellt sind.

Bei der in Fig. 6 gezeigten Drehstellung der beiden Endstücke 19, 30 liegt der maximal einstellbare Abstand C2 zwischen den beiden Abgabeöffnungen 23 und 34 (bzw. zwischen ihren Mittelachsen M1 und M3) vor, der hier 30 mm beträgt.

Bei der in Fig. 7 gezeigten Wahl der Drehstellungen der beiden Endstücke 19, 30 liegt ein Abstand C3 zwischen den beiden Abgabeöffnungen 23 und 34 vor, der zwischen den beiden Abständen C1 und C2 liegt.

In Fig. 8 sind Drehstellungen der Endstücke 19, 30 gezeigt, bei denen die Drehstellung nicht spiegelsymmetrisch zu einer Mittelebene ist, die zwischen den beiden Spritzenzylindern 3 und 4 liegt und sich vom oberen Ende 11 zum unteren Ende 13 erstreckt (eine Ebene parallel zur Zeichenebene gemäß Fig. 2).

Durch die erfindungsgemäße Ausbildung der Spritze 1 und insbesondere der Endstücke 19 und 30 in Verbindung mit den Adaptern 18 und 29 kann somit der Abstand der Abgabeöffnungen 23 und 34 nach Bedarf verändert und eingestellt werden.

Die Endstücke 19 und 30 sind insbesondere so ausgebildet, das Injektionskanülen (nicht gezeigt) vorgesehen werden können. Durch die Verstellung des Abstandes der beiden Abgabeöffnungen 23 und 34 wird dann auch gleichzeitig der Abstand der Injektionskanülen geändert und eingestellt.

Bei der beschriebenen Ausführungsform ist die erfindungsgemäße Spritze 1 als Selbstfüllerspritze ausgebildet. Dies muss nicht sein. Sie kann auch als Spritze 1 ausgebildet sein, die keine Selbstfüllerspritze ist.

Bei der beschriebenen Ausführungsform sind die beiden Spritzenzylinder 3 und 4 baugleich ausgebildet. Dies ist nicht zwingend notwendig. Beispielsweise kann der Innendurchmesser des ersten Spritzenzylinders 3 verschieden sein vom Innendurchmesser des zweiten Spritzenzylinders 4. Die Spritzenzylinder 3 und 4 können Spritzenhohlräume 7 und 10 mit einem Volumen von z.B. 1,0 - 5,0 ml aufweisen.

Ferner ist es auch möglich, mehr als zwei Spritzenzylinder 3, 4 vorzusehen, wenn dies gewünscht ist.

Die erfindungsgemäße Spritze ist insbesondere so ausgebildet, dass der maximale Kolbenhub und somit die Dosierung pro Ausspritzvorgang für jeden der beiden Kolben 5 und 8 voneinander unabhängig einstellbar ist. So kann der Kolbenhub für beide Kolben 5, 8 gleich oder verschieden sein.

## Patentansprüche

1. Spritze mit
einem ersten Spritzenzylinder (3), der ein erstes Abgabeende (15), an dem ein erstes Endstück (19) mit einem ersten Kanal (22) befestigt ist, aufweist,
einem verschiebbar im ersten Spritzenzylinder (3) angeordneten ersten Kolben (5), der zum ersten Abgabeende (15) verschiebbar ist, um ein zwischen dem ersten Abgabeende (15) und dem ersten Kolben (5) befindliches Fluid über den ersten Kanal (22) abzugeben, einem zweiten Spritzenzylinder (4), der ein zweites Abgabeende (26), an dem ein zweites Endstück (30) mit einem zweiten Kanal (33) befestigt ist, aufweist, und
einem verschiebbar im zweiten Spritzenzylinder (4) angeordneten zweiten Kolben (8), der zum zweiten Abgabeende (26) verschiebbar ist, um ein zwischen dem zweiten Abgabeende (26) und dem zweiten Kolben (8) befindliches Fluid über den zweiten Kanal (32) abzugeben,
wobei die beiden Spritzenzylinder (3, 4) mechanisch so miteinander verbunden sind, dass ihr Abstand nicht änderbar ist,
***dadurch gekennzeichnet, dass***
zumindest eines der beiden Endstücke (19, 30) zur Verstellung des Abstandes der beiden Kanäle (22, 33) in unterschiedlichen Stellungen am zugeordneten Abgabeende (15, 26) befestigbar ist,
und wobei das zumindest eine der beiden Endstücke (19, 30) in unterschiedlichen Drehstellungen um eine Mittelachse des Endstücks (19, 30) am zugeordneten Abgabeende (15, 26) befestigbar ist, wobei der Kanal (22, 33) des zumindest einen der beiden Endstücke (19, 30) exzentrisch zur Mittelachse (M1, M2) des Endstücks (19, 30) angeordnet ist.

2. Spritze nach Anspruch 1, bei der das zumindest eine der beiden Endstücke (19, 30) ein zum zugeordneten Spritzenzylinder (3, 4) zugewandtes Ende (40, 41) aufweist, das in eine am Abgabeende (15, 26) des zugeordneten Spritzenzylinders (3, 4) vorgesehene Aufnahme (36, 37) in zumindest zwei unterschiedlichen Drehstellungen einsetzbar ist.

3. Spritze nach Anspruch 2, bei der eine Fixiereinrichtung vorgesehen ist, die eine Fixierung des zumindest einen der beiden Endstücke (19, 30) in der gewählten Drehstellung bewirkt.

4. Spritze nach Anspruch 2 oder 3, bei der die Aufnahme (26, 38) hohlzylinderförmig und das entsprechende Ende (40, 41) zylinderförmig ist.

5. Spritze nach Anspruch 4, bei der die hohlzylinderförmige Ausnehmung (36, 37) eine Kontur mit n Kanten und das Endstück (40, 41) eine Kontur mit m Kanten aufweist, wobei n und m ganze Zahlen sind und n größer oder gleich m ist.

6. Spritze nach einem der obigen Ansprüche, bei der das zumindest eine der beiden Endstücke (19, 30) mittels einer Überwurfmutter (20, 31) am zugeordneten Abgabeende (15, 26) befestigt ist.

7. Spritze nach einem der obigen Ansprüche, bei der die beiden Endstücke (19, 30) so ausgebildet sind, dass an ihnen lösbar jeweils eine Hohlkanüle befestigbar ist.

8. Spritze nach einem der obigen Ansprüche, bei der die beiden Spritzenzylinder (3, 4) zueinander parallel ausgerichtet sind und die beiden Kanäle (22, 33) zueinander parallel ausgerichtet sind.

9. Spritze nach einem der obigen Ansprüche, bei der die Spritze als Selbstfüllerspritze ausgebildet ist.

10. Spritze nach einem der obigen Ansprüche, mit einem Hauptteil (2), an dem die beiden Spritzenzylinder (3, 4) befestigt sind, und einem am Hauptteil (2) angelenkten Hebel (12), der mit den Kolben (5, 8) verbunden ist, um sie bei Betätigung des Hebels (12) zu den Abgabeenden (15, 26) zu bewegen.

11. Spritze nach einem der obigen Ansprüche, bei der in zumindest einem der beiden Kolben (5, 8) ein Zuführkanal (24, 35) ausgebildet ist, der in den ersten bzw. zweiten Spritzenzylinder (3, 4) mündet.

12. Spritze nach Anspruch 11, bei der der erste Kolben (5) mit einer ersten Kolbenstange (6) verbunden ist, die über das dem ersten Abgabeende (15) abgewandte hintere Ende des ersten Spritzenzylinders (3) heraussteht,
der zweite Kolben (8) mit einer zweiten Kolbenstange (9) verbunden ist, die über das dem zweiten Abgabeende (26) abgewandte hintere Ende des zweiten Spritzenzylinders (4) heraussteht,
wobei sich der Zuführkanal (24, 35) des zumindest einen der beiden Kolben (5, 8) durch die damit verbundene Kolbenstange (6, 9) erstreckt.

13. Spritze nach Anspruch 12, bei der der in der Kolbenstange (6, 9) verlaufende Zuführkanal am von dem verbundenen Kolben (5, 8) abgewandten Ende in einen Anschluss (25) mündet, in dem ein erstes Rückschlagventil angeordnet ist, das bei einer Bewegung des Kolbens (5, 8) weg vom Abgabeende (15, 26) öffnet.

14. Spritze nach einem der obigen Ansprüche, bei dem im zumindest einem der beiden Abgabeenden (15, 26) ein zweites Rückschlagventil angeordnet ist, das bei einer Bewegung des entsprechenden Kolbens (5, 8) zum Abgabeende (15, 26) hin öffnet.

## Claims

1. Syringe with
a first syringe barrel (3) which comprises a first delivery end (15) to which a first end piece (19) with a first channel (22) is secured,
a first piston (5) which is arranged movable in the first syringe barrel (3) and can be moved towards the first delivery end (15) in order to deliver a fluid located between the first delivery end (15) and the first piston (5) via the first channel (22),
a second syringe barrel (4) which comprises a second delivery end (26) to which a second end piece (30) with a second channel (33) is secured, and
a second piston (8) which is arranged movable in the second syringe barrel (4) and can be moved towards the second delivery end (26) in order to deliver a fluid located between the second delivery end (26) and the second piston (8) via the second channel (32), wherein the two syringe barrels (3, 4) are mechanically connected to each other such that the distance between them cannot be changed,
**characterized in that**
at least one of the two end pieces (19, 30) can be secured to the allocated delivery end (15, 26) in different positions for the adjustment of the distance between the two channels (22, 33),
and wherein at least one of the two end pieces (19, 30) can be secured to the allocated delivery end (15, 26) in different rotational positions about a centre axis of the end piece (19, 30), wherein the channel (22, 23) of the at least one of the two end pieces (19, 30) is arranged eccentrically relative to the centre axis (M1, M2) of the end piece (19, 30).

2. Syringe according to claim 1, in which the at least one of the two end pieces (19, 30) comprises an end (40, 41) which faces the allocated syringe barrel (3, 4) and can be inserted in at least two different rotational positions into a receiver (36, 37) provided at the delivery end (15, 26) of the allocated syringe barrel (3, 4).

3. Syringe according to claim 2, in which a fixing device is provided which brings about a fixation of the at least one of the two end pieces (19, 30) in the chosen rotational position.

4. Syringe according to claim 2 or 3, in which the receiver (26, 38) is in the shape of a hollow cylinder and the corresponding end (40, 41) is cylindrical.

5. Syringe according to claim 4, in which the hollow cylinder-shaped recess (36, 37) has a contour with n sides and the end piece (40, 41) has a contour with m sides, wherein n and m are whole numbers and n is greater than or equal to m.

6. Syringe according to any of the above claims, in which the at least one of the two end pieces (19, 30) is secured to the allocated delivery end (15, 26) by means of a union nut (20, 31).

7. Syringe according to any of the above claims, in which the two end pieces (19, 30) are formed such that a hollow-bore needle can be detachably secured to them in each case.

8. Syringe according to any of the above claims, in which the two syringe barrels (3, 4) are aligned parallel to each other and the two channels (22, 23) are aligned parallel to each other.

9. Syringe according to any of the above claims, in which the syringe is formed as a self-filling syringe.

10. Syringe according to any of the above claims, with a main part (2), to which the two syringe barrels (3, 4) are secured, and a lever which is hinged to the main part (2) and is connected to the pistons (5,8) in order to move them towards the delivery ends (15, 26) when the lever (12) is actuated.

11. Syringe according to any of the above claims, in which a feed channel (24, 35), which opens to the first or second syringe barrel (3, 4), is formed in at least one of the both pistons (5, 8).

12. Syringe according to claim 11, wherein the first piston (5) is connected with a first piston rod (6), which extends beyond the rear end of the first syringe barrel (3) facing away from the first delivery end (15),
the second piston (8) is connected to a second piston rod (9), which extends beyond a rear end of the second syringe barrel (4) facing away from a second delivery end (26), wherein the feed channel (24, 35) of the at least one of the both pistons (5, 8) extends through the piston rod (6, 9) connected with said piston.

13. Syringe according to claim 12, wherein the feed channel, which extends through the piston rod (6, 9), opens to a connection at the end facing away from the piston (5, 8), wherein a first non-return valve is provided in the connection and opens when the piston (5, 8) is moved away from the delivery end (15, 26).

14. Syringe according to any of the above claims, wherein a second non-return valve is provided in at least one of the two delivery ends (15, 26) and opens, when the corresponding piston (5, 8) is moved towards the delivery end (15, 26).

## Revendications

1. Seringue dotée
d'un premier cylindre de seringue (3), qui comprend une première extrémité de déchargement (15), au niveau de laquelle une première pièce d'extrémité (19) dotée d'un premier canal (22) est fixée,
d'un premier piston (5) agencé de manière coulissante dans le premier cylindre de seringue (3), qui peut être amené à coulisser vers la première extrémité de déchargement (15), afin de décharger par l'intermédiaire du premier canal (22) un fluide se trouvant entre la première extrémité de déchargement (15) et le premier piston (5),
d'un deuxième cylindre de seringue (4), qui comprend une deuxième extrémité de déchargement (26), au niveau de laquelle une deuxième pièce d'extrémité (30) dotée d'un deuxième canal (33) est fixée, et
d'un deuxième piston (8) agencé de manière coulissante dans le deuxième cylindre de seringue (4), qui peut être amené à coulisser vers la deuxième extrémité de déchargement (26), afin de décharger par l'intermédiaire du deuxième canal (32) un fluide se trouvant entre la deuxième extrémité de déchargement (26) et le deuxième piston (8),
les deux cylindres de seringue (3, 4) étant reliés mécaniquement l'un à l'autre de telle sorte que leur écart ne soit pas modifiable,
***caractérisée en ce que***
au moins une des deux pièces d'extrémité (19, 30) peut être fixée à différentes positions sur l'extrémité de déchargement associée (15, 26) pour le réglage de l'écart des deux canaux (22, 33),
et l'au moins une des deux pièces d'extrémité (19, 30) pouvant être fixée à différentes positions de rotation autour d'un axe médian de la pièce d'extrémité (19, 30) sur l'extrémité de déchargement associée (15, 26), le canal (22, 33) de l'au moins une des deux pièces d'extrémité (19, 30) étant agencé excentriquement par rapport à l'axe médian (M1, M2) de la pièce d'extrémité (19, 30).

2. Seringue selon la revendication 1, dans laquelle l'au moins une des deux pièces d'extrémité (19, 30) comprend une extrémité (40, 41) tournée vers le cylindre de seringue associé (3, 4), qui peut être insérée dans un logement (36, 37) prévu à l'extrémité de déchargement (15, 26) du cylindre de seringue associé (3, 4) dans au moins deux positions de rotation différentes.

3. Seringue selon la revendication 2, dans laquelle un appareil de fixation est prévu, qui réalise une fixation de l'au moins une des deux pièces d'extrémité (19, 30) dans la position de rotation choisie.

4. Seringue selon la revendication 2 ou 3, dans laquelle le logement (26, 38) est de forme cylindrique creuse et l'extrémité correspondante (40, 41) est de forme cylindrique.

5. Seringue selon la revendication 4, dans laquelle le logement de forme cylindrique creuse (36, 37) présente un contour à n bords et la pièce d'extrémité (40, 41) présente un contour à m bords, n et m étant des nombres entiers et n étant supérieur ou égal à m.

6. Seringue selon l'une quelconque des revendications précédentes, dans laquelle l'au moins une des deux pièces d'extrémité (19, 30) est fixée à l'extrémité de déchargement associée (15, 26) au moyen d'un écrou à chapeau (20, 31).

7. Seringue selon l'une quelconque des revendications précédentes, dans laquelle les deux pièces d'extrémité (19, 30) sont configurées de telle sorte qu'une canule creuse puisse être fixée de manière amovible sur chacune d'entre elles.

8. Seringue selon l'une quelconque des revendications précédentes, dans laquelle les deux cylindres de seringue (3, 4) sont orientés parallèlement l'un à l'autre et les deux canaux (22, 33) sont orientés parallèlement l'un à l'autre.

9. Seringue selon l'une quelconque des revendications précédentes, dans laquelle la seringue est configurée sous la forme d'une seringue à remplissage automatique.

10. Seringue selon l'une quelconque des revendications précédentes, dotée d'une partie principale (2), à laquelle les deux cylindres de seringue (3, 4) sont fixés, et d'un levier (12) articulé sur la partie principale (2), qui est relié avec les pistons (5, 8), afin de déplacer ceux-ci vers les extrémités de déchargement (15, 26) lors d'un actionnement du levier (12) .

11. Seringue selon l'une quelconque des revendications précédentes, dans laquelle un canal d'alimentation (24, 35) est formé dans au moins un des deux pistons (5, 8), qui débouche dans le premier ou le deuxième cylindre de seringue (3, 4).

12. Seringue selon la revendication 11, dans laquelle le premier piston (5) est relié avec une première tige de piston (6), qui dépasse au-dessus de l'extrémité arrière, détournée de la première extrémité de déchargement (15), du premier cylindre de seringue (3), le deuxième piston (8) est relié avec une deuxième tige de piston (9), qui dépasse au-dessus de l'extrémité arrière, détournée de la deuxième extrémité de déchargement (26), du deuxième cylindre de seringue (4),
le canal d'alimentation (24, 35) de l'au moins un des deux pistons (5, 8) s'étendant à travers la tige de piston (6, 9) reliée à celui-ci.

13. Seringue selon la revendication 12, dans laquelle le canal d'alimentation s'étendant dans la tige de piston (6, 9) débouche à l'extrémité détournée du piston relié (5, 8) dans un raccordement (25), dans lequel un premier clapet anti-retour est agencé, qui s'ouvre lors d'un déplacement du piston (5, 8) à distance de l'extrémité de déchargement (15, 26).

14. Seringue selon l'une quelconque des revendications précédentes, dans laquelle un deuxième clapet anti-retour est agencé dans l'au moins une des deux extrémités de déchargement (15, 26), qui s'ouvre lors d'un déplacement du piston correspondant (5, 8) vers l'extrémité de déchargement (15, 26).
